# EUROPÄISCHE PATENTSCHRIFT

(11) **EP 3 713 848 B1**
(45) Veröffentlichungstag und Bekanntmachung des Hinweises auf die Patenterteilung: **12.03.2025**
(21) Anmeldenummer: 18803425.0
(22) Anmeldetag: 16.11.2018
(51) Int. Cl.: B65D 81/24, C07B 63/04, A23L 3/3508, A23L 3/3517, C07C 68/08, A23L 2/44, B65D 23/08

(54) **DIKOHLENSÄUREDIESTER ENTHALTENDE ALUMINIUMBEHÄLTER**
DICARBONIC ACID CONTAINING ALUMINIUM CONTAINER
RÉCIPIENT EN ALUMINIUM COMPORTANT LES DIESTERS D'ACIDE DICARBONIQUE

(30) Priorität: 20.11.2017 EP 17202540
(43) Veröffentlichungstag der Anmeldung: 30.09.2020
(73) Patentinhaber: LANXESS Deutschland GmbH, 50569 Köln (DE)
(72) Erfinder: SCHÖBBEN, Karl-Joachim, 41539 Dormagen (DE); HOFMANN, Christoph, 51061 Köln (DE); TAUPP, Marcus, 40789 Monheim (DE); VOGL, Erasmus, 51429 Bergisch-Gladbach (DE)
(86) Internationale Anmeldenummer: PCT/EP2018/081591
(87) Internationale Veröffentlichungsnummer: WO 2019/097005

(56) Entgegenhaltungen:
- WO-A1-2016/066764
- CN-U- 204 368 790
- DE-A1- 102007 045 958
- DE-A1- 102015 104 671
- JP-A- 2005 093 375
- JP-A- H05 186 404
- US-A- 3 186 906
- US-A- 3 936 269
- DATABASE WPI Week 199334, Derwent World Patents Index; AN 1993-269780
- DATABASE WPI Week 200530, Derwent World Patents Index; AN 2005-289036

## Beschreibung

Die Erfindung betrifft Dikohlensäurediester enthaltende Aluminiumbehälter und ein Verfahren zur Herstellung der verpackten Dikohlensäurediester.

Dikohlensäurediester, insbesondere Dimethyldicarbonat und Diethyldicarbonat, werden in der Getränkeindustrie zur Kaltentkeimung von alkoholfreien karbonisierten oder stillen Fruchtsaftgetränken, Fruchtsäften, Weinen, alkoholfreien Weinen, Ciders, Eis-Tees und anderen Getränken eingesetzt. Diese Technologie der Getränkestabilisierung hat eine Reihe von Vorteilen. Der herausragende Vorteil liegt dabei in der Tatsache begründet, dass Geschmack und Farbe, im Gegensatz zur Heißabfüllung, nicht beeinflusst werden. Auch gegenüber persistenten Konservierungsstoffen, wie Natriumbenzoat bzw. Benzoesäure oder Kaliumsorbat bzw. Sorbinsäure, besteht der Vorteil insbesondere in der Abwesenheit jeglicher geschmacklicher Beeinträchtigungen. Gegenüber einer kaltaseptischen Abfüllung sind besonders die ganz wesentlich geringeren Investitionskosten in Anlagentechnik als Vorteil beim Einsatz von Dikohlensäurediestern bekannt.

Andere Anwendungen von Dikohlensäurediestern, wie beispielweise die Inaktivierung von Enzymen oder die Verwendung in Batterien, sind ebenfalls beschrieben.

Die Substanzklasse der Dikohlensäurediester hat die besondere Eigenschaft in Kontakt mit entsprechenden Getränken in die abgeleiteten Alkohole und Kohlendioxid zu hydrolysieren. Typische Beispiele für Dikohlensäurediester sind Dimethyldicarbonat, Diethyldicarbonat, Di- iso-propyldicarbonat, Di-n-propyldicarbonat oder Di-tert.-butyldicarbonat.

In Abhängigkeit von der Temperatur der Getränke während der Anwendung ist deshalb schon nach relativ kurzer Zeit die eigentlich aktive Substanz nicht mehr im Getränk vorhanden. Bei den üblichen Temperaturen zwischen 5 und 20 °C ist dies nach einigen Stunden der Fall. Die Hydrolyse wird aber auch schon durch geringste Mengen von Wasser eingeleitet.

Der Zerfall von Dikohlensäurediestern ist nicht nur auf die Hydrolyse beschränkt, ein Abbau findet auch durch eine spontane Decarboxylierung statt. Dabei entsteht Kohlendioxid und Dialkylcarbonat. Da die Stabilität von Dimethyldicarbonat generell niedrig ist und grundsätzlich eine Tendenz zu Hydrolyse und Decarboxylierung besteht, sind Methoden zur Stabilisierung bei Herstellung, Lagerung und Transport von Dikohlensäurediestern beschrieben worden. Trotz der beschrieben Methoden stellt die Lagerung und der Transport ein nicht gelöstes Problem dar, denn eine unkontrollierte und unzeitgemäße Zersetzung führt zur vorzeitigen Zerstörung der Dikohlensäurediester und zu einem Druckaufbau innerhalb der Gebinde. Dies kann wiederum zu Gefahren bei der Handhabung solcher in der Zersetzung befindlicher Behälter führen.

Üblicherweise werden Dikohlensäurediester nach dem Stand der Technik in Glasgefäße abgefüllt und in Glasgefäßen transportiert. Die Stabilität der Dikohlensäurediester in Glasgefäßen ist befriedigend, stellt jedoch in der technischen Verwendung oftmals eine Einschränkung dar. Typische Haltbarkeitszeiten betragen beispielsweise für Dimethyldicarbonat für den Lebensmitteleinsatz maximal 12 Monate. Eine Verlängerung der Lagerzeiten wäre wünschenswert. Zudem ist der Einsatz von flüssigen Stoffen in Glas immer problematisch, da Glas leicht bricht und bei Glasbruch ein Auslaufen nicht verhindert werden kann. Aus diesem Grund werden teilweise auf der Außenseite polymerbeschichtete Glasgefäße eingesetzt, welche aber in der Herstellung teuer sind. Zudem wird nach dem Stand der Technik das Glasgefäß in einer Styropor-Umverpackung transportiert, was aber ebenfalls sowohl relativ teuer als auch umständlich ist.

Trotz dieser Maßnahmen muss ein Glasgebinde immer sehr sorgfältig gehandhabt werden um Glasbruch zu vermeiden, was einen großen Nachteil darstellt.

Weiter ist Glas zur Aufbewahrung für Chemikalien welche Gase abspalten können problematisch, da es durch Überdruck in den Glasgefäßen zu deren Bersten kommen kann und die umherfliegenden Glassplitter dann ein zusätzliches Sicherheitsrisiko für die Beschäftigten darstellen können. Auch kann die polymere Außenschicht der Glasgefäße durch Hitze oder mechanische Einwirkung angegriffen werden.

Als weiteres Verpackungsmaterial für Stoffe aller Art sind neben Glas auch Metallbehälter bekannt. Leider zersetzen sich Dialkyldicarbonate in allen bekannten Metallbehältern relativ schnell. Auf Grund ihres geringen Gewichtes und ihrer Korrosionsbeständigkeit sind im Lebensmittelbereich besonders Behälter aus Aluminium bekannt. Um die Beständigkeit gegenüber Lebensmitteln zu verbessern wurden eine Reihe von beschichteten Aluminiumbehälter beschrieben.

Aus der US-B2-8142858 sind beispielweise Behälter aus Aluminiummetall bekannt, die mit Epoxidharzen beschichtet sind oder ein Multischichtensystem aus Polyesterharzen, Polyvinylchlorid und Polymethacrylatharzen aufweisen. Diese mehrfachbeschichteten Behältnisse sind für den Transport und die Aufbewahrung von Nahrungsmitteln und Getränken geeignet.

Aus der US-B2-6008273 ist bekannt, dass Epoxyharze oder Phenolharze zur Bildung einer wasserfesten Schichten auf Metallflaschen, insbesondere auch Aluminiumflaschen eingesetzt werden können.

Aus der US-B-8001961 sind Aluminiumflaschen bekannt, die mit phenolischen Epoxidharzen beschichtet werden können und dann zum Transport und Lagerung von lichtempfindlichen, gasförmigen Anästhetika eingesetzt werden.

Aus der DE-A-19912794 sind Aluminiumflaschen bekannt, die mit Mischungen aus Polyesterharzen und Resolharzen beschichtet sind und im Wesentlichen frei von Bisphenol-A-diglycidylethern sind. Diese Flaschen werden für die Befüllung mit Lebensmitteln und Getränken bereitgestellt.

Die US-A-3936269 beschreibt die Abfüllung von Dimethyldicarbonat in Flaschen, Containern und Dosen mit dem Ziel, die dort enthaltenen Getränke und Nahrungsmittel zu konservieren.

Aus der US-A-3186906 ist bekannt, dass auch nicht verderbliche Gegenstände, wie Aluminiumcontainer, mit Dimethyldicarbonat sterilisiert und wieder verwendet werden können.

Die CN-U-204368790 beschreibt ein Batteriesäurereservoir, wobei die Batteriesäure aus LiPF₆ und weiteren Substanzen zusammengesetzt ist, wobei eine dieser Substanzen u.a. Dimethylcarbonat sein könnte.

Die DE-A-102007045958 beschreibt ein Verfahren zur Herstellung von mikrobiell stabilisierten Getränken, bei dem ein Dialkyldicarbonat in Form einer wässrigen und/oder organischen Lösung dem Getränk zugegeben wird. Es wurden auch Verfahren zur Behandlung der Dikohlensäurediester bekannt, die die Transport- und Lagereigenschaften verbessern sollen. So wird beispielsweise in der US-A-3936269 ein Verfahren vorgeschlagen, bei dem Dimethyldicarbonat in gefrorenem Zustand transportiert und gelagert wird und zur Verwendung wieder aufgetaut wird. In gefrorenem Zustand ist Dimethyldicarbonat deutlich stabiler und lagerfähig, aber die Handhabung ist technisch deutlich komplexer und daher mit hohen Kosten verbunden und wird in der Praxis nicht angewandt.

In den bestehenden Transport- und Lagerungssystemen kann die Stabilität von Dikohlensäurediestern nicht hinreichend gewährleistet werden.

Es bestand daher weiterhin die Aufgabe neue Transportbehältnisses für Dikohlensäurediester zu finden mit denen sich die Nachteile aus dem Stand der Technik überwinden lassen.

Überraschend wurde nun gefunden, dass Dikohlensäurediester in Aluminiumbehältern, welche auf der Innenseite mit polymeren Beschichtungen ausgestattet sind, im Wesentlichen zersetzungsfrei gelagert und transportiert werden können.

Gegenstand der Erfindung ist daher ein Füllgut enthaltender Aluminiumbehälter, wobei das Füllgut mindestens eine Verbindung der Formel (I) 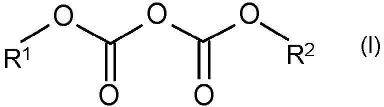 worin
R¹ und R² unabhängig voneinander für geradkettiges oder verzweigtes C₁-C₅-Alkyl stehen, und der Aluminiumbehälter mit mindestens einem Polymeren, zumindest auf der Innenseite, beschichtet ist, wobei dieses Polymer Polyester ist. Ganz besonders bevorzugt stehen R¹ und R² unabhängig voneinander für Methyl oder Ethyl.

Noch weiter bevorzugt sind die Verbindungen der Formel (I) Dimethyldicarbonat oder Diethyldicarbonat oder Mischungen daraus.

Das Füllgut stellt im Allgemeinen Verbindungen der Formel (I) oder Gemische der Verbindungen der Formel (I) dar. Das Füllgut kann ebenfalls weitere Zusätze, wie z.B. anorganische oder organische Alkali- oder Erdalkalimetallsalze, Trocknungsmittel, wie z.B. Calciumchlorid oder Silicagel oder Sauerstofffänger, wie z.B. Sulfite oder Phosphate enthalten. Bevorzugt enthält das Füllgut keine weiteren Zusätze. Der Begriff Füllgut umfasst die zu transportierenden oder/und zu lagernden Verbindungen. Der Begriff Füllgut umfasst vorzugsweise nicht die Schutzgase oder Luft, die gegebenenfalls zusätzlich in dem Aluminiumbehälter enthalten sind. Bevorzugt sind die Verbindungen der Formel (I) oder die Gemische der Verbindungen der Formel (I) in einer Menge von 90 bis 100 % bezogen auf die Gesamtmenge des Füllgutes in dem Aluminiumbehälter enthalten. Besonders bevorzugt sind die Verbindungen der Formel (I) oder die Gemische der Verbindungen der Formel (I) in einer Menge von 95 bis 100 %, ganz besonders bevorzugt in einer Menge von 98 bis 100 % bezogen auf die Gesamtmenge des Füllgutes, in dem Aluminiumbehälter enthalten. Noch weiter bevorzugt liegen die Verbindungen der Formel (I) in einer Menge von 99 bis 100 % bezogen auf die Gesamtmenge des Füllgutes, in dem Aluminiumbehälter vor.

Als Aluminiumbehälter im Sinne der Erfindung werden alle Behälter verstanden, die zumindest auf der Innenseite eine Aluminiumschicht aufweisen, die mit dem Polymeren beschichtet werden kann. Mit Innenseite im Sinne der Erfindung wird die Seite des Aluminiumbehälters verstanden, die über die Polymerbeschichtung mit dem Füllgut in Kontakt steht. Die Außenseite des Behälters kann auch aus einem anderen Metall, oder einer Legierung oder aus Kunststoff bestehen. Der Aluminiumbehälter besteht vorzugsweise vollständig aus Aluminium und besitzt eine Polymerbeschichtung auf der Innenseite. Vorzugsweise hat das Aluminium, welches mit dem Polymeren beschichtet ist, eine Reinheit > 99 %.

Der Aluminiumbehälter ist mit mindestens einem Polymeren, zumindest auf der Innenseite, beschichtet, wobei dieses Polymer Polyester ist. Der Aluminiumbehälter kann mit weiteren Polymeren beschichtet sein.

Bei den Polymeren im Sinne der Erfindung handelt es sich bevorzugt um Polyester, Phenolplaste, Acrylharze, Aminoplaste oder Epoxidharze, die auch als Mischungen eingesetzt werden können.

Verschiedene kommerziell erhältliche Polyester sind zur Verwendung in der vorliegenden Erfindung geeignet. Vorzugsweise werden als Polyester VITE^{®} Polyester (z.B. VITE^{®} PE-100 und PE-200 gesättigte Polyester erhältlich von Goodyear Tire and Rubber Co., Akron, Ohio), URALAC^{®} Polyester (z.B. URALAC^{®} ZW5000SH, URALAC^{®} SN978, URALAC^{®} SN908 erhältlich von Royal DSM N.V.) und Dynapol^{®} Polyester (z.B. Dynapol^{®} LH and L, gesättigte Polyesterharze, erhältlich von Evonik Industries AG, Marl) eingesetzt. Besonders bevorzugt wird URALAC^{®} XP 8481 SN (Royal DSM N.V.) als Polyester eingesetzt.

Vorzugsweise weist der Polyester ein mittleres Molekulargewicht (MW) von 500 bis etwa 10.000 g/mol, besonders bevorzugt von 1000 bis 7500 g/mol auf. Die Glasübergangstemperatur (Tg) des Polyesters ist vorzugsweise größer oder gleich 50 °C, besonders bevorzugt größer oder gleich 60 °C. Vorzugsweise ist die Glasübergangstemperatur des Polyester kleiner oder gleich 100 ° C.

Der Begriff Polyester umfasst Polyester, die aus gleichen oder verschiedenen, also gemischten unterschiedlichen Monomeren, hergestellt werden können. Der Polyester wird typischerweise aus Polycarbonsäuren oder Anhydriden oder Mischungen daraus und Polyolen hergestellt. Die Herstellung des Polyesters erfolgt nach bekannten Kondensations- bzw. Veresterungsverfahren, wie sie z.B. in Zeno Wicks, Jr., Frank N. Jones und S. Peter Pappas, Organic Coatings: Wissenschaft und Technologie , Vol. 1, S. 122-132 (John Wiley and Sons: New York, 1992), beschrieben werden.

Bevorzugt werden als Polycarbonsäuren zur Herstellung der Polyester Maleinsäure, Fumarsäure, Bernsteinsäure, Adipinsäure, Phthalsäure, 5-tert.-butyl-isophthalic Säure, Azelainsäure, Sebacinsäure, Chlorendic-Säure, Isophthalsäure, Trimellitsäure Terephthalsäure, Methyltetrahydrophthalsäure, Methylhexahydrophthalsäure, Tetrahydrophthalsäure, Dodecandisäure, Azelainsäure, Naphthalindicarbonsäure, Pyromellitsäure und Dimerfettsäuren, Cyclohexandicarbonsäure, wie insbesondere 1,4-Cyclohexandicarbonsäure, Glutarsäure, 12-Hydroxystearinsäure, 2-Hydroxypropionsäure und 2-Hydroxybuttersäure und Mischungen daraus eingesetzt. Besonders bevorzugt werden Dicarbonsäuren, wie insbesondere, Phthalsäure, Isophthalsäure, Terephthalsäure, 1,4-Cyclohexandicarbonsäure, Bernsteinsäure, Sebacinsäure, Methyltetrahydrophthalsäure, Methylhexahydrophthalsäure, Tetrahydrophthalsäure, Dodecandisäure, Adipinsäure, Azelainsäure, Naphthalindicarbonsäure, Pyromellitsäure und Dimerfettsäuren eingesetzt.

Bevorzugt werden als Anhydride Bernsteinsäureanhydrid, Maleinsäureanhydrid, Phthalsäureanhydrid, Tetrahydrophthalsäureanhydrid, Hexahydrophthalsäureanhydrid, Endomethylentetrahydrophthalsäureanhydrid, Tetrachlorphthalsäureanhydrid, Trimellitanhydrid, Pyromellitdianhydrid sowie die Säureanhydride, der bevorzugt eingesetzten Polycarbonsäuren und deren Niederalkylester, wie insbesondere deren Methylester eingesetzt. Bevorzugt werden als Niederalkylester der Polycarbonsäuren Terephthalsäuredimethylester, Bernsteinsäuredimethylester und Adipinsäuredimethylester eingesetzt.

Als Polyole können vorzugsweise Ethylenglykol, Propylenglykol, 1,2- und 1,3-Propandiol, Diethylenglykol, Dipropylenglykol, Triethylenglykol, Tetraethylenglykol, 1,4-Butandiol, 1,3-Butylethylpropanediol, 2-Methyl-1,3-propandiol, 1,5-Pentandiol, Cyclohexandimethanol, Tricyclodecanedimethanol, Glycerin, 1,6-Hexandiol, Neopentylglykol, Pentaerythrit, Trimethylolethan, Trimethylolpropan, 1,4-Benzyldimethanol und -ethanol, 2,4-Dimethyl-2-ethylhexan-1,3-diol, ein Polyethylen- oder Polypropylenglykol mit einem mittleren Molekulargewicht (Mw) kleiner oder gleich 500 g/mol, Isopropyliden-bis (p-phenylenoxypropanol-2) und Mischungen davon eingesetzt werden. Als Polyole werden besonders bevorzugt Diole eingesetzt. Bevorzugte Diole sind Ethylenglykol, Propylenglykol, Diethylenglykol, Neopentylglykol und Gemische davon.

Besonders bevorzugt werden Trimethylolpropan, Neopentylglycol, Tricyclodecanedimethanol oder 1,4-Butandiol oder Mischungen davon als Polyole eingesetzt.

Vorzugsweise weist das eingesetzte Polyester Hydroxy- oder Carboxylgruppen als Endfunktionalitäten auf. Besonders bevorzugt weist der eingesetzte Polyester Hydroxygruppen als Endfunktionalitäten auf. Dies wird üblicherweise durch die Verwendung einer überschüssigen Menge an Polyolen während der Veresterungsreaktion erreicht.

Als Polymere im Sinne der Erfindung können auch Phenolplaste eingesetzt werden. Der Begriff Phenolplaste umfasst Phenolplaste, die aus gleichen oder verschiedenen, also gemischten unterschiedlichen Monomeren, hergestellt wurden. Als Phenoplaste werden die Kondensationsprodukte von Aldehyden mit Phenolen eingesetzt. Vorzugsweise werden als Aldehyde Formaldehyd und Acetaldehyd eingesetzt. Vorzugsweise werden als Phenole Xylenol, Kresol, p-Phenylphenol, p-tert.-Butylphenol, p-tert.-Amylphenol und Cyclopentylphenol. Vorzugsweise enthalten die einsetzbaren Phenolplaste mindestens zwei oder mehr reaktive Hydroxylgruppen als funktionelle Gruppen, damit die Phenolplaste mit den Polyestern vernetzt werden können. Vorzugsweise sind die Phenolplaste im Wesentlichen frei von Bisphenol A (BPA), Bisphenol F (BPF), Bisphenol-A-diglycidylehter (2,2'-bis(4-hydroxyphenyl)propan-bis(2,3-epoxypropyl)ether) (BADGE) und Bisphenol-F-diglycidylether (BFDGE).

Im Sinne der Erfindung bedeutet der Begriff "im Wesentlichen frei von", dass der Gehalt an den genannten Verbindungen < 5 ppm ist.

Besonders bevorzugt werden als Phenolplaste mit Butanol veretherte Phenolplaste eingesetzt.

Vorzugsweise werden als Phenolplaste handelsübliche Phenolplaste wie insbesondere, DUREZ^{®} und VARCUM^{™} von DUREZ Corp. ((Dallas, Tex.) oder Reichhold Chemical AG (Österreich); (CO) POLYMEROX^{™} von Monsanto Chemical Co. (St. Louis, MO); Arofene^{™} und Arotap^{™} von Ashland Chemical Co. (Dublin, Ohio); und BAKELITE^{®} von Bakelite A. G. (Iserlohn, Deutschland) oder Phenodur^{®} PR 899, oder Phenodur^{®} PR515 oder Phenodur^{®} PR516 (Allnex GmbH) oder Mischungen diese Phenolplaste eingesetzt.

Besonders bevorzugt werden als Phenolplaste BAKELITE PF 6470 LB^{®}, Bakelit 9989LB^{®}, VARCUM 2227 B55^{®}, Phenodur^{®} PR 899, Phenodur^{®} PR515 oder Phenodur^{®} PR516 oder Mischungen aus diesen Phenolplasten eingesetzt.

Als weitere Polymere im Sinne der Erfindung können auch Epoxidharze verwendet werden. Vorzugsweise werden als Epoxidharze, die Reaktionsprodukte aus der Umsetzung von Phenolen mit Epoxide eingesetzt. Besonders bevorzugt werden die Epoxidharze aus der Umsetzung von Bisphenol A, Bisphenol F, Phenol, Butylphenol, Xylenol- und Kresol mit Epichlorhydrin eingesetzt. Noch weiter bevorzugt werden als Epoxidharze Epikote^{®} 828 der Fa. Hexion GmbH, Deutschland eingesetzt.

Als weitere Polymere können auch Polyacrylate eingesetzt werden. Polyacrylate im Sinne der Erfindung sind vorzugsweise Polymethacrylate oder Polyacrylate der Acrylsäure zu verstehen. Vorzugsweise enthalten diese Polyacrylate funktionelle Gruppen, wie vorzugsweise Carboxyl, Hydroxy oder Oxirangruppen. Noch weiter bevorzugt enthalten die Polyacrylate, mindestens eine Carboxylgruppe oder eine Oxiran-funktionelle Gruppe, gegebenenfalls in Kombination mit einem oder mehreren Hydroxylgruppen.

In einer bevorzugten Ausführungsform der Erfindung werden Polyacrylate mit einem mittleren Molekulargewicht (MW) von 1.000 bis 50.000 g/mol, bevorzugt von 2.000 bis 25.000 g/mol und besonders bevorzugt von 5.000 bis 10.000 g/mol, eingesetzt. Die Glasübergangstemperatur der Polyacrylate liegt vorzugsweise im Bereich von -24 °C bis 105 °C und weiter bevorzugt im Bereich von 50 °C bis 90 °C.

Vorzugsweise ist das Polyacrylat ein Copolymer aus Methacrylsäure (MA) und Acrylsäure (AA) mit Ethylmethacrylat und Butylmethacrylat oder ein Copolymer aus 2-Hydroxyethylmethacrylat (HEMA) mit Ethylmethacrylat oder ein Copolymer aus Glycidylmethacrylat (GMA) mit Ethylmethacrylat oder ein Copolymer aus Glycidylmethacrylat mit Hydroxypropylmethacrylat und Styrol.

Das Polyacrylat wird nach bekannten chemische Syntheseverfahren, zum Beispiel durch Polymerisation von ethylenisch ungesättigten Acrylmonomeren mittels radikalischer Polymerisation hergestellt.

In einer weiteren Ausführungsform der Erfindung stellen die Polymere Aminoplaste dar. Aminoplaste schließen die Kondensationsprodukte von Aldehyden, wie Formaldehyd, Acetaldehyd, Crotonaldehyd und Benzaldehyd mit Verbindungen, die Amino- oder Amidogruppe enthalten, wie Harnstoff, Melamin und Benzoguanidin ein. Vorzugsweise werden als Aminoplaste, Aminoplaste mit zwei oder mehr Aminogruppen als funktionelle Gruppen eingesetzt.

Geeignete Aminoplaste sind vorzugsweise Benzoguanidin-Formaldehyd Polymerisate, Melamin-Formaldehyd Polymerisate, esterfizierte Melamin-Formaldehyd Polymere und Harnstoff-Formaldehyd Polymere. Vorzugsweise wird als Aminoplast das vollständig alkylierte Melamin-Formaldehyd Polymer, welches im Handel von Cytec Industries (Cytec Industries GmbH, Neuss, Deutschland) unter dem Handelsnamen CYMEL^{®} 303 erhältlich ist, eingesetzt.

In einer weiteren Ausführungsform der Erfindung sind die Polymere vernetzt. Jeder geeignete Hydroxyl-, Amino-, Vinyl- oder Isocyanatgruppen enthaltender Vernetzer kann verwendet werden. In einer bevorzugten Ausführungsform der Erfindung dienen die Polymere, wie insbesondere Aminoplaste, Phenolplaste oder Epoxidharze, als Vernetzer.

In einer weiteren Ausführungsform der Erfindung können die Polymere ebenfalls durch Isocyanatgruppen vernetzt werden. Vorzugsweise werden als Isocyanate aliphatische und cycloaliphatische Polyisocyanate, wie vorzugsweise Hexamethylendiisocyanat (HDI), Isophorondiisocyanat (IPDI), Bis[4-isocyanatocyclohexyl]methan (TMXDI), Tetramethylenm-xylidindiisocyanat (H 12 MDI), Isopropenyldimethylbenzylisocyanat (TMI), Dimeren oder Trimeren davon und Mischungen davon eingesetzt. Geeignete kommerziell erhältliche Isocyanat-Vernetzer sind vorzugsweise VESTANAT^{®} B 1358 A, VESTANAT^{®} EP B 1186 A, VESTANAT^{®} EP B 1299 SV (Evonik Resource Efficiency GmbH, Marl, Deutschland) und DESMODUR^{®} BL 3175 (Bayer AG, Leverkusen, Deutschland).

Die Polymere können ohne weitere Hilfsmittel eingesetzt werden, insbesondere wenn die Polymere bei Raumtemperatur und unter Normaldruck flüssig sind, oder mit weiteren organischen Lösungsmitteln, Schmiermitteln, Katalysatoren, Pigmenten und Additiven vermischt werden. Im Rahmen dieser Erfindung werden als Polymerzusammensetzungen, Polymere in An- oder Abwesenheit weiterer organischer Lösungsmittel, Schmiermitteln, Katalysatoren, Pigmenten und Additiven, bezeichnet. Bevorzugt sind Polymerzusammensetzungen, die Polymere in Gegenwart weiterer organischer Lösungsmittel, Schmiermitteln, Katalysatoren, Pigmenten und/oder Additiven enthalten.

Als organische Lösungsmittel, die besonders nützlich als Trägerflüssigkeiten für die Polymere sind, werden vorzugsweise aliphatische Kohlenwasserstoffe, wie beispielsweise und vorzugsweise Lösungsbenzin, Kerosin und P Naphtha; aromatische Kohlenwasserstoffe, wie insbesondere, Benzol, Toluol, Xylol oder Solventnaphtha 100, 150, 200 (Exxon Chemicals GmbH); Alkohole, wie insbesondere, Ethanol n-Propanol, Isopropanol, n-Butanol oder iso-Butanol; Ketone wie insbesondere 2-Butanon, Cyclohexanon, Methyl-Arylketone, Ethyl-Arylketone oder Methyl-Isoamyl-Ketone; Ester, wie insbesondere Ethylacetat oder Butylacetat; Glykole, wie insbesondere Butylglykol, Glykolether, wie insbesondere Methoxypropanol; Glykolether, wie insbesondere Ethylenglykolmonomethylether, Ethylenglykolmonoethylether, Ethylenglykolmonobutylether, Propylenglykolmonomethylether; Glycolester, wie insbesondere Butylglykolacetat oder Methoxypropylacetat, oder Mischungen davon, eingesetzt. Besonders bevorzugt wird Solventnaptha 100, 150 oder 200 oder Mischungen davon eingesetzt. Solventnaphta ist auch unter dem Handelsnamen Solvesso^{™} bekannt.

Katalysatoren können eingesetzt werden, um die Geschwindigkeit der Härtung oder Vernetzung zu erhöhen.

Vorzugsweise werden als Katalysatoren quaternäre Ammoniumverbindungen, Phosphorverbindungen, Zinn- und Zinkverbindungen, wie vorzugsweise Tetraalkylammonium Halogenid, ein Tetraalkylammonium oder Tetraarylphosphoniumiodid oder -acetat, Zinnoctoat, Zinkoctoat, Triphenylphosphin oder Mischungen davon eingesetzt.

Besonders bevorzugt werden als Katalysatoren Phosphor- Säureester-Lösungen, wie vorzugsweise ADDITOL XK 406^{™} (Cytec Surface Specialties, Inc., West Paterson, NJ) oder Sulfonsäuren, wie vorzugsweise CYCAT 600^{™} (Cytec Surface Specialties, Inc., West Paterson Dodecylbenzol, NJ) oder Mono- und Di-octyl-zinnmercaptide, wie vorzugsweise Tinstab OTS 17 MS^{™} (AKZO-Nobel Chemicals, Inc., Chicago, III.) oder Dibutylzinndilaurat, wie vorzugsweise FASCAT^{™} (Atofina Chemicals, Inc., Philadelphia, Pa.) oder Mischungen dieser Katalysatoren eingesetzt.

In einer bevorzugten Ausführungsform wird der Katalysator in einer Menge von 0,05 Gew.% bis 5 Gew.%, bevorzugt in einer Menge von 0,1 bis 1,5 Gew.% bezogen auf das Gewicht der Polymerzusammensetzung eingesetzt.

Die Polymerzusammensetzungen können Schmiermittel enthalten. Vorzugsweise werden als Schmiermittel langkettige aliphatische Wachse, wie insbesondere Carnauba-Wachse (Luba-Print 887 / C-Wachs-Dispersion, Münzing Chemie GmbH, Abstatt, Deutschland) synthetische Wachsdispersionen, wie vorzugsweise Lanco^{™} GLIDD 4518V (Lubrizol, Corp., Wickliffe, Ohio), Lanco^{™} Wax TF 1780 EF, Lanco^{™} Wax 1350 FF (Lubrizol, Corp., Wickliffe, Ohio), Polyethylen, Polypropylen, Lanolin, Polytetrafluorethylen und Mischungen dieser Verbindungen eingesetzt.

Es können auch Pigmente der Polymerzusammensetzung zugesetzt werden. Geeignete Pigmente, wie Aluminiumflocken, Titandioxid und Zinkoxid werden üblicherweise verwendet um das Aussehen der Schutzschicht zu verbessern. Werden Aluminiumflocken als Pigmente eingesetzt, werden diese vorzugsweise in einer Menge von 2 Gew.% bis 15 Gew.% und besonders bevorzugt in einer Menge von 5 Gew.% bis 10 Gew.%, bezogen auf Gewicht der Polymerzusammensetzung eingesetzt. Ein Pigment wie Titandioxid, wird vorzugsweise in einer Menge von 35 Gew.% bis 50 Gew.% und noch weiter bevorzugt in einer Menge von 40 Gew.% bis 45 Gew.%, bezogen auf das Gewicht der Polymerzusammensetzung, eingesetzt werden. Wird Zinkoxid als Pigment eingesetzt liegt dieses vorzugsweise in einer Menge von 0,5 Gew.% bis 30 Gew.%, und noch weiter bevorzugt in einer Menge von 5 Gew.% bis 15 Gew.% bezogen auf das Gewicht der Polymerzusammensetzung, vor.

Besonders bevorzugt ist die Menge an Pigmenten in der Polymerzusammensetzung kleiner 300 ppm. Ganz besonders bevorzugt ist die Menge an Pigmenten in der Polymerzusammensetzung kleiner 50 ppm. Noch weiter bevorzugt enthält die Polymerzusammensetzung keine Pigmente.

Es ist von der jeweiligen Anwendung abhängig, ob die Polymerzusammensetzung weitere Additive, wie Wasser, Tenside, Dispergiermittel (vorzugsweise Lecithin), Entschäumer (vorzugsweise modifizierte Polysiloxane), Verdickungsmittel (vorzugsweise Methylcellulose), oder Füllstoffe, wie vorzugsweise Silikate oder Aluminium-/ Magnesiumhydroxide sowie Magnesiumoxid, Kalziumoxid oder Ruß oder Mischungen davon, enthalten kann.

Besonders bevorzugt ist die Menge an Füllstoffen in der Polymerzusammensetzung kleiner 300 ppm. Ganz besonders bevorzugt ist die Menge an Füllstoffen in der Polymerzusammensetzung kleiner 50 ppm Noch weiter bevorzugt enthält die Polymerzusammensetzung keine Füllstoffe.

Die Polymerzusammensetzung wird vorzugsweise durch einfaches Vermischen vom Polymer und aller wahlweisen Bestandteile in jeder gewünschten Reihenfolge unter ausreichendem Rühren hergestellt. Die resultierende Mischung wird vorzugsweise gemischt, bis alle Bestandteile der Zusammensetzung im Wesentlichen homogen vermischt sind. Vorzugsweise wird die Polymerzusammensetzung bei Temperaturen zwischen 10 °C und 50 °C hergestellt.

Die Polymerzusammensetzung wird vorzugsweise in flüssiger Form als Lösung, Suspension oder Dispersion auf die Aluminiumoberfläche aufgebracht. Dies kann mittels Streichen, Eintauchen (Dipping), Rollen oder Besprühen geschehen oder mittels vergleichbarer Verfahren. Bevorzugt wird die Suspension durch Besprühen auf die Aluminiumoberfläche des Behälters aufgetragen.

Wenn sie als eine flüssige Beschichtung aufgebracht wird, weist die Polymerzusammensetzung vorzugsweise einen Feststoffgehalt von 25 Gew.% bis 70 Gew.% nicht-volatiles Material und besonders bevorzugt von 30 Gew.% bis 50 Gew.% nicht-volatiles Material auf.

In einer bevorzugten Ausführungsform enthält die Polymerzusammensetzung 20 Gew.% bis 80 Gew.% Polyester und 1 Gew. % bis 80 Gew.% organische Lösungsmittel und weniger als 1 Gew.% Schmiermittel und/oder Katalysatoren.

In einer weiteren bevorzugten Ausführungsform enthält die Polymerzusammensetzung 20 Gew. % bis 75 Gew. % Polyester, 10 Gew.% bis 79 Gew.% organische Lösungsmittel und 1 Gew. % bis 25 Gew. % andere Polymere, wie insbesondere Polyacrylate, Epoxidharze, Phenolplaste oder Aminoplaste und weniger als 1 Gew.% Schmiermittel und/oder Katalysatoren.

In einer weiteren bevorzugten Ausführungsform enthält die Polymerzusammensetzung 20 Gew. % bis 75 Gew. % Polyester, 10 Gew.% bis 79 Gew.% organische Lösungsmittel und 1 Gew. % bis 25 Gew. % Phenolplaste und weniger als 1 Gew.% Schmiermittel und/oder Katalysatoren.

In einer weiteren bevorzugten Ausführungsform enthält die Polymerzusammensetzung 20 Gew. % bis 75 Gew. % Polyester, 10 Gew.% bis 79 Gew.% organische Lösungsmittel und 0,9 Gew. % bis 25 Gew. % Phenolplaste und 0 bis 2 Gew.% Katalysatoren und 0,1 bis 2 Gew.% Schmiermittel.

In einer weiteren bevorzugten Ausführungsform enthält die Polymerzusammensetzung 20 Gew. % bis 75 Gew. % Polyester, 10 Gew.% bis 79 Gew.% organische Lösungsmittel und 0,9 Gew. % bis 25 Gew. % Phenolplaste und 0 bis 2 Gew.% Katalysatoren und 0,1 bis 2 Gew.% Schmiermittel und weniger als 5 ppm Füllstoffe und/oder Pigmente.

In einer weiteren bevorzugten Ausführungsform ist der Polyester zumindest auf Basis von Pyromellitdianhydrid, Tricyclodecanedimethanol und dem Vernetzer Isophorondiisocyanat hergestellt worden. Dieser Polyester und der Vernetzer ist insbesondere in den Produkten Goldlack 32S23MC und Goldlack BT651B der Fa. Valspar Corp. Minneapolis, MN, USA enthalten. Aluminiumbehälter, die mit diesem Lack beschichtet sind und bei denen das Füllgut mit dem Goldlack 32S23MC oder/und Goldlack BT651B in Kontakt stand, lassen sich leicht mit wässrigen Lösungen reinigen. Der Lack löst sich bei diesem Reinigungsprozess von der Aluminiumoberfläche ab.

Der Härtungsprozess der Polymerzusammensetzung auf der Oberfläche wird vorzugsweise durch Erwärmung, vorzugsweise bei einer Temperatur von 150 °C bis 280 °C, durchgeführt. Die Dauer der Erwärmung liegt vorzugsweise bei 1 min bis 10 min.

Die Polymerzusammensetzungen können nach dem Fachmann bekannten Verfahren, wie z.B. durch das sogenannte Blatt-Bake-Verfahren mittels Walzenbeschichtung, dem Coil Coating Verfahren oder durch Bandbeschichtung oder auch durch Sprühverfahren im technischen Maßstab auf Aluminiumbleche aufgetragen werden. Die Polymerzusammensetzung wird dann durch Einwirkung von Wärme, aktinischer Strahlung, z.B. mittels ultraviolettem oder infrarotem Licht, durch elektromagnetische Strahlung, z.B. Elektronenstrahlhärtung oder mittels einer Kombinationen dieser Verfahren gehärtet. Die beschichteten Bleche können dann zu Behältern geformt werden. Es ist allerdings ebenfalls möglich und auch bevorzugt, die fertigen Aluminiumbehälter mit der Polymerzusammensetzung, vorzugsweise durch Besprühen, zu beschichten. Vorzugsweise wird durch Erwärmung, die Polymerzusammensetzung auf der Oberfläche gehärtet.

Die auf der Aluminiumoberfläche gebildeten Polymerbeschichtungen haben vorzugsweise eine Dicke von 1 bis 50 µm. Besonders bevorzugt haben die Polymerbeschichtungen eine Dicke von 5 bis 10 µm.

Der Rahmen der Erfindung erfasst alle oben stehenden und im Folgenden aufgeführten allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Die Erfindung umfasst ebenfalls ein Verfahren zur Herstellung des vorher beschriebenen erfindungsgemäßen Füllgut enthaltenden Aluminiumbehälters enthaltend mindestens eine Verbindung der Formel (I) 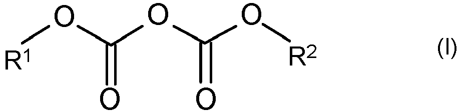 worin R¹ und unabhängig voneinander für geradkettiges oder verzweigtes C₁-C₅-Alkyl, steht, bei dem in einem Schritt a.) ein Aluminiumbehälter mit einer Polymerzusammensetzung enthaltend mindestens ein Polyester beschichtet wird und diese Schicht in einem Schritt b.) gehärtet wird und dann in einem Schritt c.) das Füllgut, gegebenenfalls unter Schutzgas, in diesen Aluminiumbehälter eingefüllt wird.

Das Füllgut wird vorzugsweise durch einen Füllstutzen in den Innenraum des Aluminiumbehälters eingefüllt. Bevorzugt erfolgt dieser Prozess des Abfüllens unter Schutzgas. Als Schutzgase kommen beispielsweise Argon, Helium oder Stickstoff in Frage. Bevorzugt wird als Schutzgas Stickstoff eingesetzt. Nach der Befüllung wird die Öffnung vorzugsweise durch eine Kunsttoffkappe, beispielsweise erhältlich von der Fa. Bericap in Budenheim, DE, verschlossen. Die Kunststoffkappen können weitere Einlagen, wie beispielsweise Dichtungsscheiben auf Basis von Polytretrafluoroethylen (PTFE), enthalten. Eine derartige Einlage ist unter der Bezeichnung "Plytrax 100" kommerziell von Norton Performance Plastic Corporation, Wayne, NJ, USA erhältlich. Bei diesem Modell wird die PTFE Schicht in der Kappe mit Hilfe eines Polyethylen-Schaums an der Kappe befestigt.

Die Lagerung und der Transport des Füllgutes kann im Allgemeinen bei Temperaturen und Drücken erfolgen, die dem Fachmann als geeignet erscheinen. Bevorzugt erfolgt die Lagerung und der Transport bei Temperaturen zwischen -78 °C und +40 °C, besonders bevorzugt bei Temperaturen zwischen -38 °C und +38 °C und ganz besonders bevorzugt bei -25 °C und + 25 °C. Bevorzugt erfolgt die Lagerung und der Transport des Füllgutes bei Atmosphärendruck, das heißt, dass kein besonderer Druck vorgegeben wird. Die Lagerung und der Transport des Füllgutes kann in Anwesenheit oder Abwesenheit eines Schutzgases erfolgen. Bevorzugt erfolgt die Lagerung und der Transport unter Anwesenheit eines Schutzgases. Vorzugsweise wird Stickstoff als Schutzgas verwendet.

Durch die speziellen, beschichteten Aluminiumbehälter können die Verbindungen der Formel (I) in einer Reinheit von mehr als 97 % über einen Zeitraum von einem bis drei Jahren gelagert werden. Besonders bevorzugt können die Verbindungen der Formel (I) in einer Reinheit von mehr als 98 % über einen Zeitraum von bis zu 2 Jahren gelagert werden. Ganz besonders können die Verbindungen der Formel (I) in einer Reinheit von mehr als 99 % über einen Zeitraum von bis zu 2 Jahren gelagert werden.

Der Füllgut enthaltende Aluminiumbehälter wird vorzugsweise an handelsübliche Dosierpumpensysteme angeschlossen. Dadurch wird es ermöglicht das Füllgut in einfacher Weise in andere Behälter, die vorzugsweise Getränke enthalten, oder Getränkelinien, einzudosieren. Daher ist von der Erfindung ebenfalls die Verwendung des Füllgut enthaltenden Aluminiumbehälters in Dosierpumpensystemen zur Umfüllung und Dosierung in andere Behälter oder Getränkelinien umfasst.

Unter der erfindungsgemäßen Verwendung der Aluminiumbehälter lassen sich die Verbindungen der Formel (I) ohne wesentliche Zersetzungen transportieren und lagern. In den bisher üblichen Verpackungen, wie z.B. Glasgebinden, die zum Transport verwendet werden, ist im Gegensatz hierzu ein erheblicher Abbau an dem aktiven Wirkstoff in kurzer Zeit festzustellen. Da das Gewicht von Aluminiumbehältern im Vergleich zu Glasgefäßen deutlich geringer ist, können die Transportkosten der Verbindungen der Formel (I) durch die erfindungsgemäße Verwendung der Aluminiumbehälter reduziert werden. Zudem sind die Gebinde nicht durch Hitze verformbar und schützen den Inhalt sicher vor Lichteinwirkung. Die erfindungsgemäß, verwendeten Aluminiumgefäße können ebenfalls recycelt werden.

### Beispiele

### Beispiel 1

### Herstellung des beschichteten Aluminiumbehälters

Ein Polyesterharzlack (Goldlack BT651B (Fa.Valspar Corp.)) wurde durch Sprühen auf die Aluminiumoberfläche, insbesondere auf die dem Füllgut zugewandte Oberfläche aufgetragen. Der Behälter wurde dann 10 min auf 190 °C erwärmt. Danach wurde optisch die Gleichförmigkeit der Beschichtung geprüft.

### Beispiel 2

### Befüllung des Aluminiumbehälters mit Dimethyldicarbonat

Die beschichteten Aluminiumflaschen aus Beispiel 1 werden mit Dimethyldicarbonat befüllt und direkt mittels einer Kunsttoffkappen von der Fa. Bericap, Budenheim, verschlossen. Die Kappen weisen eine Polytretrafluoroethylen (PTFE) Dichtung auf. Die Dichtung ist unter der Bezeichnung "Plytrax 100" kommerziell erhältlich und wurde von Norton Performance Plastic Corporation, Wayne, NJ, USA bezogen.

### Beispiel 3

### Stabilitätstests von Dimethyldicarbonat in verschiedenen Gebinden

3 kg Dimethyldicarbonat wurde in verschiedene, in der Tabelle 1 aufgelistete, kommerziell erhältliche und experimentell hergestellte Gebinde gefüllt, danach verschlossen und zur Lagerung in verschiedene Temperaturschränke gestellt. Die Bedingungen, also die Lagerdauer und die Lagertemperatur, unter denen das Füllgut in den Gebinden gelagert wurde, können der Tabelle 1 entnommen werden. Die Analytik wurde mittels Gaschromatographie durchgeführt; hierbei wurde die vorhandene Menge an Dimethylcarbonat bestimmt, das ein Zerfallsprodukt der thermischen Zersetzung von Dimethyldicarbonat ist. Wird eine hohe Menge an Dimethylcarbonat gefunden, steht dies für eine geringe Stabilität des Dimethyldicarbonates.

**Tabelle 1**

| | Lagertemperatur [°C] | Gehalt an Dimethylcarbonat [ppm]/nach X Tagen Lagerung |
|---|---|---|
| Aluminium unbeschichtet (Fa. Leicht und Appelt, Bad Gandersheim, GER) | 20 | 6724 ppm / 35 Tage |
| Aluminium eloxiert (Fa. Leicht und Appelt, Bad Gandersheim, GER) | 20 | 4462 ppm/ 35 Tage |
| Glas | 20/40/20* | 19591 ppm/ 168 Tage |
| Polyesterharzlack auf Aluminium beschichtet mit einem Pigmentgehalt von kleiner 300 ppm. (Goldlack BT651B (Fa Valspar Corp. AG, Grüningen, CH) | 20/40/20* | 120 ppm/ 365 Tage |
| Polyesterharzlack auf Aluminium beschichtet mit einem Pigmentgehalt von kleiner 300 ppm. (Goldlack 32S23MC (Valspar Corp. AG, Grüningen, CH) | 20/40/20* | 840 ppm/ 365 Tage |

| | | |
|---|---|---|
| *5 Wochen Lagerung bei 20 °C, dann 4 Wochen bei 40 °C, dann weiter gelagert bei 20°C | | |

Wie der Tabelle 1 zu entnehmen ist, ist Dimethyldicarbonat, in einem mit Polyesterlack beschichteten Aluminiumbehältern, deutlich länger lagerbar also stabiler, als in unbeschichteten oder eloxierten Aluminiumbehältern.

### Zusammenfassung

Die Erfindung betrifft Dikohlensäurediester enthaltende Aluminiumbehälter und ein Verfahren zur Herstellung der verpackten Dikohlensäurediester.

## Patentansprüche

1. Füllgut enthaltende Aluminiumbehälter, **dadurch gekennzeichnet, dass** das Füllgut mindestens eine Verbindung der Formel (I) ist 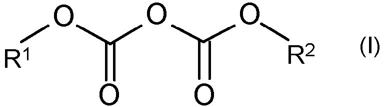 worin
R¹ und unabhängig voneinander für geradkettiges oder verzweigtes C₁-C₅-Alkyl steht,
und der Aluminiumbehälter mit mindestens einem Polymeren, zumindest auf der Innenseite, beschichtet ist, wobei dieses Polymer Polyester ist.

2. Füllgut enthaltende Aluminiumbehälter gemäß Anspruch 1, **dadurch gekennzeichnet, dass** die Verbindungen der Formel (I) Dimethyldicarbonat oder Diethyldicarbonat oder Mischungen daraus sind.

3. Füllgut enthaltende Aluminiumbehälter nach Anspruch 1 oder 2, **dadurch gekennzeichnet, dass** die Verbindungen der Formel (I) in einer Menge von 90 bis 100 %, vorzugsweise in einer Menge von 95 % bis 100 %, bezogen auf die Gesamtmenge des Füllgutes, als Füllgut vorliegt.

4. Füllgut enthaltende Aluminiumbehälter nach einem oder mehreren der Ansprüche 1 bis 3, **dadurch gekennzeichnet, dass** die Menge an Füllstoffe und/oder Pigmente kleiner oder gleich 50 ppm in der Polymerbeschichtung ist.

5. Füllgut enthaltende Aluminiumbehälter nach einem oder mehreren der Ansprüche 1 bis 3, **dadurch gekennzeichnet, dass** die Dicke der Polymerschicht 5 bis 10 µm beträgt.

6. Füllgut enthaltende Aluminiumbehälter nach Anspruch 1, **dadurch gekennzeichnet, dass** das Polymer ein Polyester mit einem mittleren Molekulargewicht (MW) von 1000 bis 7500 g/mol ist.

7. Füllgut enthaltende Aluminiumbehälter nach Anspruch 1, **dadurch gekennzeichnet, dass** das Polymer ein Polyester mit einer Glasübergangstemperatur kleiner oder gleich 100 °C ist.

8. Füllgut enthaltende Aluminiumbehälter nach Anspruch 1, **dadurch gekennzeichnet, dass** das Polymer ein Polyester ist, dass zumindest aus Pyromellitdianhydrid, Tricyclodecanedimethanol und dem Vernetzer Isophorondiisocyanat hergestellt wurde.

9. Verfahren zur Herstellung eines Füllgut enthaltenden Aluminiumbehälters nach Anspruch 1 enthaltend mindestens einer Verbindung der Formel (I) 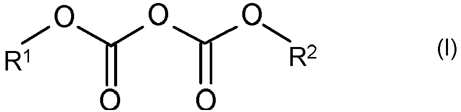 worin
R¹ und unabhängig voneinander für geradkettiges oder verzweigtes C₁-C₅-Alkyl, steht,
**dadurch gekennzeichnet, dass** in einem Schritt a.) ein Aluminiumbehälter mit einer Polymerzusammensetzung enthaltend mindestens ein Polyester beschichtet wird und diese Schicht in einem Schritt b.) gehärtet wird und dann in einem Schritt c.) das Füllgut, gegebenenfalls unter Schutzgas, in diesen Aluminiumbehälter eingefüllt wird.

10. Verfahren zur Herstellung eines Füllgut enthaltenden Aluminiumbehälters gemäß Anspruch 9 **dadurch gekennzeichnet, dass** als Polymerzusammensetzung in Schritt a.) eine Mischung enthaltend 20 Gew. % bis 75 Gew. % Polyester, 10 Gew.% bis 79 Gew.% organische Lösungsmittel und 0.9 Gew. % bis 25 Gew. % Phenolplaste und 0.1 bis 2 Gew.% Schmiermittel und weniger als 5 ppm Füllstoffe und/oder Pigmente eingesetzt wird.

11. Verfahren zur Herstellung eines Füllgut enthaltenden Aluminiumbehälters gemäß Anspruch 10, **dadurch gekennzeichnet**, das die Polymerzusammensetzung in Schritt a.) durch Besprühen auf die Aluminiumoberfläche des Behälters aufgetragen.

12. Verfahren zur Herstellung eines Füllgut enthaltenden Aluminiumbehälters gemäß einem der Ansprüche 10 oder 11, **dadurch gekennzeichnet, dass** Schritt b.) durch Erwärmung bei einer Temperatur von 150 °C bis 280 °C durchgeführt wird.

13. Verwendung der Füllgut enthaltenden Aluminiumbehälter gemäß Anspruch 1 in Dosierpumpensystemen zur Umfüllung und Dosierung des Füllgutes in andere Behälter oder Getränkelinien.

## Claims

1. Aluminium containers containing filled product, **characterized in that** the filled product is at least one compound of the formula (I) 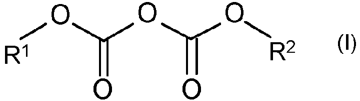 in which
R¹ and independently of one another are straight-chain or branched C₁-C₅ alkyl,
and the aluminium container is coated with at least one polymer at least on the inside, this polymer being polyester.

2. Aluminium containers containing filled product, according to Claim 1, **characterized in that** the compounds of the formula (I) are dimethyl dicarbonate or diethyl dicarbonate or mixtures thereof.

3. Aluminium containers containing filled product, according to Claim 1 or 2, **characterized in that** the compounds of the formula (I) are present as filled product in an amount of 90 to 100%, preferably in an amount of 95% to 100%, based on the total amount of the filled product.

4. Aluminium containers containing filled product, according to one or more of Claims 1 to 3, **characterized in that** the amount of fillers and/or pigments is less than or equal to 50 ppm in the polymer coating.

5. Aluminium containers containing filled product, according to one or more of Claims 1 to 3, **characterized in that** the thickness of the polymer layer is 5 to 10 µm.

6. Aluminium containers containing filled product, according to Claim 1, **characterized in that** the polymer is a polyester having an average molecular weight (MW) of 1000 to 7500 g/mol.

7. Aluminium containers containing filled product, according to Claim 1, **characterized in that** the polymer is a polyester having a glass transition temperature of less than or equal to 100°C.

8. Aluminium containers containing filled product, according to Claim 1, **characterized in that** the polymer is a polyester which has been prepared at least from pyromellitic dianhydride, tricyclodecanedimethanol and the crosslinker isophorone diisocyanate.

9. Method for producing an aluminium container containing filled product, according to Claim 1, containing at least one compound of the formula (I) 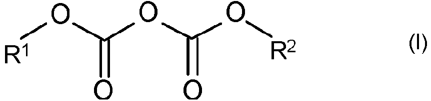 in which
R¹ and independently of one another are straight-chain or branched C₁-C₅ alkyl,
**characterized in that** in a step a.) an aluminium container is coated with a polymer composition containing at least one polyester and in a step b.) this layer is cured and then in a step c.) the product, optionally under inert gas, is filled into this aluminium container.

10. Method for producing an aluminium container containing filled product, according to Claim 9, **characterized in that** use is made as polymer composition in step a.) of a mixture containing 20 wt% to 75 wt% of polyester, 10 wt% to 79 wt% of organic solvents and 0.9 wt% to 25 wt% of phenolic resins and 0.1 to 2 wt% of lubricants, and less than 5 ppm of fillers and/or pigments.

11. Method for producing an aluminium container containing filled product, according to Claim 10, **characterized in that** the polymer composition in step a.) is applied by spraying to the aluminium surface of the container.

12. Method for producing an aluminium container containing filled product, according to one of Claims 10 and 11, **characterized in that** step b.) is carried out by heating at a temperature of 150°C to 280°C.

13. Use of the aluminium containers containing filled product, according to Claim 1, in metering pump systems for transferring and metering the product into other containers or beverage lines.

## Revendications

1. Récipient en aluminium contenant un produit de remplissage, **caractérisé en ce que** le produit de remplissage est au moins un composé de formule (I) 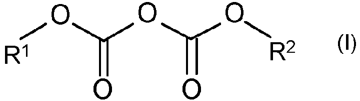 dans laquelle
R¹ et représentent indépendamment l'un de l'autre un groupe alkyle en C₁-C₅ linéaire ou ramifié,
et le récipient en aluminium est revêtu d'au moins un polymère, au moins sur le côté intérieur, ce polymère étant un polyester.

2. Récipient en aluminium contenant un produit de remplissage selon la revendication 1, **caractérisé en ce que** les composés de formule (I) sont le dicarbonate de diméthyle ou le dicarbonate de diéthyle ou des mélanges de ceux-ci.

3. Récipient en aluminium contenant un produit de remplissage selon la revendication 1 ou 2, **caractérisé en ce que** les composés de formule (I) sont présents en tant que produit de remplissage en une quantité de 90 à 100 %, de préférence en une quantité de 95 % à 100 %, par rapport à la quantité totale du produit de remplissage.

4. Récipient en aluminium contenant un produit de remplissage selon une ou plusieurs des revendications 1 à 3, **caractérisé en ce que** la quantité de charges et/ou de pigments est inférieure ou égale à 50 ppm dans le revêtement polymère.

5. Récipient en aluminium contenant un produit de remplissage selon une ou plusieurs des revendications 1 à 3, **caractérisé en ce que** l'épaisseur de la couche de polymère est de 5 à 10 µm.

6. Récipient en aluminium contenant un produit de remplissage selon la revendication 1, **caractérisé en ce que** le polymère est un polyester ayant une masse moléculaire moyenne (MW) de 1 000 à 7 500 g/mol.

7. Récipient en aluminium contenant un produit de remplissage selon la revendication 1, **caractérisé en ce que** le polymère est un polyester ayant une température de transition vitreuse inférieure ou égale à 100 °C.

8. Récipient en aluminium contenant un produit de remplissage selon la revendication 1, **caractérisé en ce que** le polymère est un polyester préparé à partir au moins de dianhydride pyromellitique, de tricyclodécanediméthanol et de l'agent de réticulation diisocyanate d'isophorone.

9. Procédé de fabrication d'un récipient en aluminium contenant un produit de remplissage selon la revendication 1, contenant au moins un composé de formule (I) 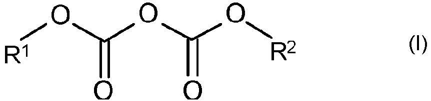 dans laquelle
R¹ et représentent indépendamment l'un de l'autre un groupe alkyle en C₁-C₅ linéaire ou ramifié,
**caractérisé en ce que**, dans une étape a.), un récipient en aluminium est revêtu d'une composition de polymère contenant au moins un polyester et cette couche est durcie dans une étape b.) et ensuite, dans une étape c.), le produit de remplissage est introduit dans ce récipient en aluminium, éventuellement sous gaz protecteur.

10. Procédé de fabrication d'un récipient en aluminium contenant un produit de remplissage selon la revendication 9, **caractérisé en ce qu'**en tant que composition de polymère dans l'étape a.), on utilise un mélange contenant 20 % en poids à 75 % en poids de polyester, 10 % en poids à 79 % en poids de solvants organiques et 0,9 % en poids à 25 % en poids de résines phénoliques et 0,1 à 2 % en poids de lubrifiant et moins de 5 ppm de charges et/ou de pigments.

11. Procédé de fabrication d'un récipient en aluminium contenant un produit de remplissage selon la revendication 10, **caractérisé en ce que** la composition de polymère est appliquée par pulvérisation sur la surface en aluminium du récipient dans l'étape a).

12. Procédé de fabrication d'un récipient en aluminium contenant un produit de remplissage selon l'une quelconque des revendications 10 ou 11, **caractérisé en ce que** l'étape b.) est réalisée par chauffage à une température de 150 °C à 280 °C.

13. Utilisation des récipients en aluminium contenant le produit de remplissage selon la revendication 1 dans des systèmes de pompe doseuse pour le transvasement et le dosage du produit de remplissage dans d'autres récipients ou lignes de boissons.
